# EUROPÄISCHE PATENTSCHRIFT

(11) **EP 3 720 542 B1**
(45) Veröffentlichungstag und Bekanntmachung des Hinweises auf die Patenterteilung: **31.07.2024**
(21) Anmeldenummer: 18815152.6
(22) Anmeldetag: 03.12.2018
(51) Int. Cl.: A61M 39/22, F16K 11/085, F16K 27/06, F16K 5/04

(54) **KÜKENHAHN FÜR DIE MEDIZINTECHNIK**
PLUG VALVE FOR MEDICAL TECHNOLOGY
ROBINET À SOUPAPE POUR LA TECHNIQUE MÉDICALE

(30) Priorität: 06.12.2017 DE 102017129033
(43) Veröffentlichungstag der Anmeldung: 14.10.2020
(73) Patentinhaber: Hopf, Hans-Jürgen, 90513 Zirndorf (DE)
(72) Erfinder: HOPF, Michael, 90513 Zirndorf (DE); KASSAI, Norbert, 90522 Oberasbach (DE)
(74) Vertreter: Ipside
(86) Internationale Anmeldenummer: PCT/EP2018/083373
(87) Internationale Veröffentlichungsnummer: WO 2019/110522

(56) Entgegenhaltungen:
- WO-A1-2011/119021
- WO-A2-2006/025054
- WO-A2-2011/048204
- DE-A1- 102015 119 899
- GB-A- 1 218 955
- US-A- 3 783 900
- US-A1- 2006 033 066

## Beschreibung

Die vorliegende Erfindung betrifft ein Verbindungssystem, insbesondere mit einem Kükenhahn, wie es beispielsweise in der Medizin und/oder Medizintechnik eingesetzt wird.

Verbindungssysteme im genannten Sinn sind im Stand der Technik bekannt. Diese werden vorzugsweise als Konnektoren für enterale und/oder parenterale Anwendungen verwendet. In zumindest einigen dieser Anwendungen können auch höhere Drücke, z.B. im Bereich von bis zu 3 bar, in Einzelfällen sogar bis zu 10 bar, auftreten. Diese Verbindungssysteme sollen eine hohe Dichtigkeit im gesperrten Zustand aufweisen, eine hohe Durchflussmenge und/oder -geschwindigkeit im geöffneten Zustand ermöglichen und dabei leichtgängig bedienbar sein. Verbindungssysteme im Stand der Technik verwenden zu diesem Zweck relativ dickwandige Ausführungen des Kükens, die häufig am Boden des Verbindungssystems abgestützt werden. Diese Vorrichtungen weisen unter anderem die Nachteile auf, dass sie bei höheren Drücken nicht dicht genug sind oder schwergängig bedienbar sind.

Ausgehend von diesem Stand der Technik ist es Aufgabe der vorliegenden Erfindung, die Nachteile des Stands der Technik wenigstens teilweise zu überwinden bzw. zu verbessern.

Die Aufgabe wird durch eine Vorrichtung gemäß Anspruch 1 gelöst. Bevorzugte Ausführungsformen und Abwandlungen sind Gegenstand der Unteransprüche.

Ein erfindungsgemäßes Verbindungssystem für fluiddurchströmte Bauteile für die Medizin und/oder Medizintechnik umfasst ein Stellglied und ein Grundgehäuse. Das Stellglied weist einen zylindrischen Abschnitt mit einer Außenfläche auf. An einem ersten Ende des zylindrischen Abschnitts ist vorzugsweise ein Griff angeordnet. Im Bereich eines zweiten Endes weist der zylindrische Abschnitt einen Hohlraum auf, derzum zweiten Ende hin offen ist, und im Bereich des zweiten Endessind mindestens zwei torförmige Durchlassöffnungen für eine Fluidverbindung angeordnet. Das Grundgehäuse weist einen Grundkörper und mindestens zwei seitlich an dem Grundkörper angeordnete Fluidpassagen auf. Der Grundkörper weist einen Hohlraum mit einem offenen ersten Ende, einem Boden im Bereich eines zweiten Endes und mindestens zwei seitlich angeordneten Öffnungen auf, welche jeweils zu den korrespondierenden Fluidpassagen eine Fluidverbindung herstellen. Der Hohlraum nimmt durch das offene erste Ende den zylindrischen Abschnitt des Stellglieds drehbeweglich auf, und der Innendurchmesser des Hohlraums entspricht dem Außendurchmesser des zylindrischen Abschnitts des Stellglieds. Die seitlich angeordneten Öffnungen korrespondieren mit den Durchlassöffnungen des Stellglieds.

Die Erfindung ist dadurch gekennzeichnet, dass an dem Boden des Grundkörpers ein, im Wesentlichen zylindrischer, Vorsprung angeordnet ist und im Bereich des zweiten Endes des Stellglieds eine umlaufende Kehlung angeordnet ist, welche den Vorsprung zumindest teilweise umgreift und damit ein Gegenlager für das zweite Ende des Stellglieds bildet.

Ein Stellglied ist ein Element, das so ausgestaltet ist, dass es in einem Verbindungssystem beweglich angeordnet ist. Diese Beweglichkeit sollte dabei die Dichtigkeit des Verbindungssystems nicht beeinträchtigen. Ein erfindungsgemäßes Stellglied ist dafür geeignet, in einen Grundkörper eingepasst zu werden. Dafür weist das Stellglied einen zylindrischen Abschnitt auf, mit einer Außenfläche, deren Durchmesser etwa dem Innendurchmesser eines Hohlraums in dem Grundkörper entspricht, so dass eine gute Passung der beiden Elemente - und somit eine gute Beweglichkeit und die Dichtigkeit des Verbindungssystems - gewährleistet ist.

Der Griff, der an dem ersten Ende des Stellglieds angeordnet ist, kann z.B. eine Art Querstange bilden, so dass das Stellglied ein T-förmiges Aussehen bekommt. Der Griff kann, insbesondere bei einer Ausführungsform des Verbindungssystems als Dreiwegehahn, auch drei Ausbuchtungen oder Äste aufweisen. Der Griff und das Stellglied kann einstückig ausgeführt sein. Der Griff kann auch nur aus einer Aufnehmung bestehen, beispielsweise in Form einer Inbus-Buchse, in welche ein geeignetes Griffelement eingepasst werden kann.

Der zylindrische Abschnitt des Stellglieds weist, zumindest teilweise, einen Hohlraum auf, so dass das zweite Ende des Stellglieds offen ist. Von diesem zweiten Ende erstrecken sich Durchlassöffnungen, die vorzugsweise torförmig sind, d.h. die Form eines umgedrehten U aufweisen. Andere Formen sind auch möglich; bevorzugt weist die Durchlassöffnung Formen auf, die mit den - seitlich am Grundkörper angeordneten - Öffnungen des Grundgehäuses korrespondieren. Damit ist durchgängig ein möglichst großer Durchmesser der Durchflussstrecke gewährleistet. Die Durchflussstrecke umfasst die Fluidpassagen des Grundkörpers, deren Öffnungen und die Durchlassöffnungen des Stellglieds. Damit das Stellglied auch einem höheren Druck eines Fluids widerstehen kann, wird das zweite Ende des Stellglieds abgestützt, und zwar durch einen, im Wesentlichen zylindrischen, Vorsprung oder Zapfen an dem Boden des Grundkörpers. Damit auch an dieser Stelle nur eine geringe Reibung auftritt, ist im Bereich des zweiten Endes des Stellglieds eine umlaufende Kehlung angeordnet, welche den Vorsprung zumindest teilweise umgreift und damit ein Gegenlager für das zweite Ende des Stellglieds bildet.

Durch diese Gestaltung des Verbindungssystems, insbesondere durch die Einführung und die besondere Ausgestaltung des Gegenlagers, erreicht ein erfindungsgemäßes Verbindungssystem eine hohe Dichtigkeit, auch bei höherem Druck, und weist dennoch eine geringe Reibung und damit eine leichtgängige Bedienbarkeit auf. Dabei ist ein großer Durchmesser der gesamten Durchflussstrecke gegeben, so dass eine hohe Durchflussmenge und/oder -geschwindigkeit im geöffneten Zustand ermöglicht wird.

Erfindungsgemäß ist die Oberkante des Vorsprungs am Boden des Grundkörpers nicht höher als die Unterkante einer der Fluidpassagen des Grundkörpers.

Dadurch, dass die Oberkante des Vorsprungs, in Bezug auf den Boden des Grundkörpers, nicht höher ist als die Unterkante einer der Fluidpassagen, ist durchgängig - d.h. auch innerhalb der Passage durch das Stellglied - ein großer Durchmesser der Durchflussstrecke gewährleistet. Damit wird der Durchfluss durch das gesamte Verbindungssystem vorteilhafterweise weiter verbessert.

In einigen Ausführungsformen weist der Vorsprung einen Hohlraum auf.

Damit wird nicht nur die Strömung des Fluids im Verbindungssystem positiv beeinflusst, sondern es ergibt sich damitauch noch eine Materialeinsparung. Der Hohlraum des Vorsprungs kann nach außen oder - bevorzugt - nach innen weisen.

In einigen Ausführungsformen weist die der Vorsprung Durchbrüche auf. Dies ist für Ausführungsformen, bei denen der Hohlraum des Vorsprungs nach innen weist.

Dies ist sowohl für solche Ausführungsformen vorteilhaft, bei denen der Vorsprung massiv gestaltet ist, als auch fürsolche, bei denen der Vorsprung einen Hohlraum aufweist; denn auch hierdurch wird der Durchfluss weiter verbessert. Wenn derVorsprung massiv gestaltet ist, dann sind diese Durchbrüche als Schlitz gestaltet; bei drei und mehr Öffnungen als verzweigter Schlitz.

Die Oberkante des Vorsprungs ist bevorzugt rund oder abgeschrägt ausgeführt. Die umlaufende Kehlung des Stellglieds, die mit dem Vorsprung korrespondiert, ist dabei ebenfalls abgeschrägt oder abgerundet.

In einigen Ausführungsformen weist die Fluidpassage bzw. deren Mantel im Bereich ihrer Außenöffnung ein Innengewinde, ein Außengewinde oder eine andere geeignete Verbindungsstruktur auf, um als Anschlussstelle für ein fluiddurchströmtes Bauteil, insbesondere für die Medizin und/oder Medizintechnik, zu fungieren. In vielen Fällen sind die Fluidpassage bzw. deren Mantel so gestaltet, dass sie mit z.B. mit einem Luer-Anschluss und/oder mit einem Anschluss gemäß ENFit^{™} (insbesondere gemäß ISO 80369-3) zusammenpassen.

In einigen Ausführungsformen verbleibt, wenn der zylindrische Abschnitt des Stellglieds in dem Grundkörper des Grundgehäuses angeordnet ist, ein Abstand zwischen dem zweiten Ende des Stellglieds und dem Boden des Hohlraums, so dass ein ringförmiger Freiraum gebildet wird.

Dadurch, dass das zweite Ende des zylindrischen Abschnitts des Stellglieds von dem Boden des Hohlraums beabstandet ist, entsteht vorteilhafterweise ein ringförmiger Freiraum, durch den das Fluid fließen kann und den Durchfluss damit weiter verbessert. Auch führt diese Ausführung zu einer weiteren Reduktion der Reibung zwischen dem Stellglied und dem Grundkörper.

In einigen Ausführungsformen weist der zylindrische Abschnitt des Stellglieds eine ringförmige Ausformung und der Grundkörper des Grundgehäuses eine radial umlaufende Aussparung auf, so dass, wenn der zylindrische Abschnitt des Stellglieds in dem Grundkörper des Grundgehäuses angeordnet ist, die ringförmige Ausformung in die radial umlaufende Aussparung einrastet.

Dies bietet den Vorteil einer besseren Dichtigkeit des Verbindungssystems. Weiterhin wird das Stellglied gegen Herausfallen geschützt oder dagegen, dass es bei hohem Druck aus dem Grundkörper gepresst wird. Diese Ausführungsformen eignen sich insbesondere auch für höhere Drücke. Es können auch mehrere ringförmige Ausformung und entsprechen mehrere radial umlaufende Aussparungen vorhanden sein.

In einer Ausführungsform weist der zylindrische Abschnitt im Bereich eines ersten Endes einen weiteren Hohlraum auf, der von dem Hohlraum getrennt ist.

Dies hat die Vorteile einer Materialeinsparung und einer höheren Elastizität des Stellglieds.

In einer Ausführungsform weist das Verbindungssystem im Bereich des zweiten Endes des zylindrischen Abschnitts, zwischen den Durchlassöffnungen einen Vorsprung und/oder ein Rücksprung auf, der sich horizontal oder vertikal erstreckt. Dieser korrespondiert mit einem Rücksprung und/oder Vorsprung des Zapfens. Damit kann ein Hahn besonders einfach und mit sensorischer Rückkopplung in seine vorgesehenen oder bevorzugten Stellungen einrasten.

In einigen Ausführungsformen weist das Verbindungssystem folgende Kombinationen der Anzahl der Öffnungen des Grundkörpers und der Anzahl der Öffnungen des zylindrischen Abschnitts auf: 2 und 2, oder3 und 2, oder3 und 3, oder4 und 2, oder4 und 3, oder4 und 4.

In einigen Ausführungsformen weisen die torförmigen Durchlassöffnungen des Stellglieds einen halbrunden, insbesondere U-förmigen, rechteckigen oder dreieckigen Abschnitt auf. Die Durchlassöffnungen können auch sechseckig etc. oder unsymmetrisch sein. Vorzugsweise sind die Formen der Durchlassöffnungen an die Formen der Öffnungen der Fluidpassagen des Grundkörpers angepasst.

In einigen Ausführungsformen werden die mit dem Fluid in Kontakt kommenden Oberflächen des Verbindungssystems wenigstens abschnittsweise aus einem amorphen Copolyester hergestellt.

In einigen Ausführungsformen werden neben dem Copolyester weitere Materialien verwendet, welche aus einer Gruppe ausgewählt werden, welche duro- und thermoplastische Kunststoff und insbesondere Polyphenylensulfid, Polypropylen, Poly-1-buten, 10 Polyvinylchlorid, Polyvinylidenchlorid, Polymethyl-metaacrylat, Polyacrylnitril, Polystyrol, Polysulfon, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff- Harz, Melamin-Harz, Alkylharz, Allylharz, Silicon, Polyimid, Polybenzimidazol, Epoxidharz, Casein-Kunststoff, vernetztes Polyurethan, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen, und/oder Kombinationen hiervon umfasst.

Ein erfindungsgemäßes Verbindungssystem wird bevorzugt für die Medizintechnik verwendet.

Die Erfindung wird nachfolgend anhand verschiedener Ausführungsformen erläutert, wobei darauf hingewiesen wird, dass durch dieses Beispiel Abwandlungen beziehungsweise Ergänzungen, wie sie sich für den Fachmann unmittelbar ergeben, mit umfasst sind. Darüber hinaus stellt dieses bevorzugte Ausführungsbeispiel keine Beschränkung der Erfindung in derArt dar, dass Abwandlungen und Ergänzungen im Umfang der vorliegenden Erfindung liegen.

Dabei zeigen:
- **Fig. 1:**: Eine perspektivische Darstellung eines erfindungsgemäßen Verbindungssystems;
- **Fig. 2:**: eine Schnittdarstellung durch ein erfindungsgemäßes Verbindungssystem.

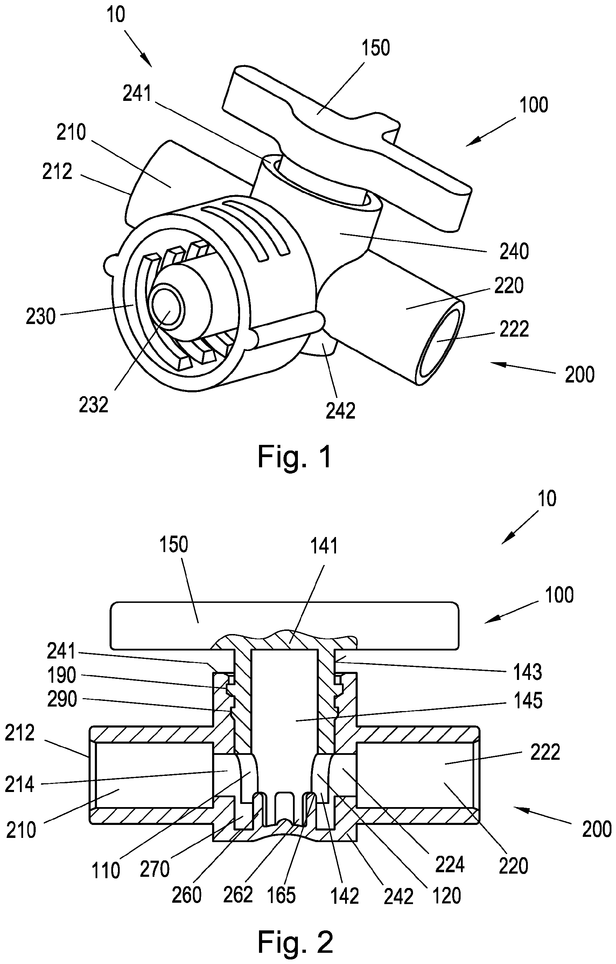

**Fig.** 1 zeigt ein Verbindungssystem 10, bei dem der zylindrische Abschnitt 140 des Stellglieds 100 in dem Grundkörper 240 des Grundgehäuses 200 angeordnet ist. Der zylindrische Abschnitt 140 des Stellglieds 100 ist deshalb nur teilweise sichtbar. An dem ersten Ende 141 des zylindrischen Abschnitts 140 ist ein Griff 150 angeordnet. In der Mitte des Grundgehäuses 200 ist der Grundkörper 240 mit einem offenen ersten Ende 241 und einem geschlossenen zweiten Ende 242 sichtbar. Weiterhin sind die Mäntel der Fluidpassagen 210, 220, 230 und deren Außenöffnungen 212, 222, 232 sichtbar.

Die Art des Zusammenwirkens des Stellglieds 100 mit dem Grundgehäuse 200 wird besonders aus der Schnittdarstellung in **Fig. 2** deutlich. Die Durchflussstrecke erstreckt sich horizontal von den Außenöffnungen 212, 222, durch die Fluidpassagen 210, 220 des Grundgehäuses 200, durch die torförmigen Durchlassöffnungen 110, 120 des Stellglieds 100 und durch einen Teil des Hohlraum 145 des Stellglieds 100. Dabei ist klar ersichtlich, dass die Form der torförmige Durchlassöffnungen 110, 120 mit den Öffnungen 214, 224 in der Wand des Grundkörpers 240 korrespondieren. DerVorsprung 260 weist einen Hohlraum 265 und Durchbrüche 262 auf. Die Oberkante des Vorsprungs 260 ist nicht höher als die Unterkante einer der Fluidpassagen 210, 220. Diese Ausgestaltungen wirken so zusammen, dass bei der gezeigten Ausführungsform ein Fluid recht ungehindert durch die gesamte Durchflussstrecke fließen kann. Das zweite Ende 142 des Stellglieds 100 ist vom Boden des Grundgehäuses 200 beabstandet, so dass ein Freiraum 270 gebildet wird, durch den das Fluid ebenfalls fließen kann. DerVorsprung 260 und die korrespondierende umlaufende Kehlung 165 sind abgerundet. Die geringe Berührungsfläche dieser Elemente trägt zur Reduzierung der Reibung zwischen Stellglied 100 und Grundgehäuse 200 bei. Oberhalb der torförmigen Durchlassöffnungen 110, 120 sind zwei radial umlaufende Aussparungen 290 an dem Grundkörper 240 angeordnet, die mit Ausformungen 190, die sich umlaufend aus der Außenfläche 143 des Stellglieds 100 hervorheben, korrespondieren. Diese Elemente erhöhen die Dichtigkeit des Verbindungssystems 10, so dass auch bei höheren Drücken kein Fluid austreten kann. Gleichzeitig trägt dies weiter dazu bei, die Reibung zu reduzieren, weil deswegen keine überaus enge Passung zwischen der Außenfläche 143 des Stellglieds 100 und dem Grundkörper 240 erforderlich ist.

### Liste der Bezugszeichen

- 10: Verbindungssystem
- 100: Stellglied
- 110, 120, 130: torförmige Durchlassöffnung
- 140: zylindrischer Abschnitt
- 141: erstes Ende
- 142: zweites Ende
- 143: Außenfläche
- 145: Hohlraum
- 147: weiterer Hohlraum
- 150: Bedienelement, Griff
- 160: Stirnfläche
- 165: umlaufende Kehlung
- 190: Ausformung
- 200: Grundgehäuse
- 210,220,230: Fluidpassage
- 212,222,232: Außenöffnung
- 214,224,234: Öffnung
- 240: Grundkörper
- 241: erstes Ende des Grundkörpers
- 242: zweites Ende, Boden, des Grundkörpers
- 245: Hohlraum des Grundkörpers
- 260: Vorsprung, (durchbrochener) Zapfen
- 262: Durchbruch
- 265: Hohlraum
- 270: Freiraum
- 290: radial umlaufende Aussparung

## Patentansprüche

1. Verbindungssystem (10) für fluiddurchströmte Bauteile für die Medizin und/oder Medizintechnik, mit
einem Stellglied (100), aufweisend einen zylindrischen Abschnitt (140) mit einer Außenfläche (143),
- wobei an einem ersten Ende (141) des zylindrischen Abschnitts (140) ein Griff (150) angeordnet ist,
- wobei der zylindrische Abschnitt (140) im Bereich eines zweiten Endes (142) einen Hohlraum (145) aufweist, der zum zweiten Ende (142) hin offen ist, und
- wobei im Bereich des zweiten Endes (142) mindestens zwei torförmige Durchlassöffnungen (110, 120, 130) für eine Fluidverbindung angeordnet sind, und
einem Grundgehäuse (200), aufweisend einen Grundkörper (240) und mindestens zwei seitlich an dem Grundkörper (240) angeordnete Fluidpassagen (210, 220, 230),
- wobei der Grundkörper (240) einen Hohlraum (245) mit einem offenen ersten Ende (241), einem Boden (242) im Bereich eines zweiten Endes und mindestens zwei seitlich angeordneten Öffnungen (214, 224, 234), welche jeweils zu den korrespondierenden Fluidpassagen (210, 220, 230) eine Fluidverbindung herstellen, aufweist,
- wobei der Hohlraum (245) durch das offene erste Ende (241) den zylindrischen Abschnitt (140) des Stellglieds (100) drehbeweglich aufnimmt und der Innendurchmesser des Hohlraums (245) dem Außendurchmesser des zylindrischen Abschnitts (140) des Stellglieds (100) entspricht, und
- wobei die seitlich angeordneten Öffnungen (214, 224, 234) mit den Durchlassöffnungen (110, 120, 130) des Stellglieds (100) korrespondieren,
**dadurch gekennzeichnet, dass**
an dem Boden (242) des Grundkörpers (240) ein, im Wesentlichen zylindrischer, Zapfen (260) zum Abstützen des zweiten Ende (142) des Stellglieds (100) angeordnet ist, und
im Bereich des zweiten Endes (142) des Stellglieds (100) eine umlaufende Kehlung (165) ausgeformt ist, welche den Zapfen (260) zumindest teilweise umgreift und damit ein Gegenlager für das zweite Ende (142) des Stellglieds (100) bildet, wobei die Kehlung (165) den Zapfen (260) berührt, und
die Oberkante des Zapfens (260) nicht höher als die Unterkante einer der Fluidpassagen (210, 220, 230) ist.

2. Verbindungssystem (10) nach Anspruch 1, **dadurch gekennzeichnet, dass** der Zapfen (260) einen Hohlraum (265) aufweist.

3. Verbindungssystem (10) nach einem der vorangehenden Ansprüche, **dadurch gekennzeichnet, dass**
der Zapfen (260) Durchbrüche (262) aufweist.

4. Verbindungssystem (10) nach einem der vorangehenden Ansprüche, **dadurch gekennzeichnet, dass**
der Zapfen (260) und die korrespondierende umlaufende Kehlung (165) zumindest teilweise abgeschrägt oder abgerundet sind.

5. Verbindungssystem (10) nach einem der vorangehenden Ansprüche, **dadurch gekennzeichnet, dass**
die Fluidpassage (210, 220, 230) des Grundgehäuses (200) im Bereich ihrer Außenöffnung (212, 222, 232) ein Innengewinde, ein Außengewinde oder eine andere geeignete Verbindungsstruktur aufweist, um als Anschlussstelle für ein fluiddurchströmtes Bauteil, insbesondere für die Medizin und/oder Medizintechnik, zu fungieren.

6. Verbindungssystem (10) nach einem der vorangehenden Ansprüche, **dadurch gekennzeichnet, dass**
wenn der zylindrische Abschnitt (140) des Stellglieds (100) in dem Grundkörper (240) des Grundgehäuses (200) angeordnet ist, ein Abstand zwischen dem zweiten Ende (142) des Stellglieds (100) und dem Boden des Hohlraums (245) verbleibt, so dass ein ringförmiger Freiraum (270) gebildet wird.

7. Verbindungssystem (10) nach einem der vorangehenden Ansprüche, **dadurch gekennzeichnet, dass**
der zylindrische Abschnitt (140) des Stellglieds (100) eine ringförmige Ausformung (190) und der Grundkörper (240) des Grundgehäuses (200) eine radial umlaufende Aussparung (290) aufweist,
so dass, wenn der zylindrische Abschnitt (140) des Stellglieds (100) in dem Grundkörper (240) des Grundgehäuses (200) angeordnet ist, die ringförmige Ausformung (190) in die radial umlaufende Aussparung (290) einrastet.

8. Verbindungssystem (10) nach einem der vorangehenden Ansprüche, **dadurch gekennzeichnet, dass**
der zylindrische Abschnitt (140) im Bereich eines ersten Endes (141) einen weiteren Hohlraum (147) aufweist, der von dem Hohlraum (145) getrennt ist.

9. Verbindungssystem (10) nach einem der vorangehenden Ansprüche, **dadurch gekennzeichnet, dass**
im Bereich des zweiten Endes (142) des zylindrischen Abschnitts (140), zwischen den Durchlassöffnungen (110, 120, 130), ein Vorsprung und/oder ein Rücksprung, der sich horizontal oder vertikal erstreckt, angeordnet ist, welcher mit einem Rücksprung und/oder Vorsprung des Zapfens (260) korrespondiert.

10. Verbindungssystem (10) nach einem der vorangehenden Ansprüche, **dadurch gekennzeichnet, dass**
das Verbindungssystem (10) vorzugsweise folgende Kombinationen der Anzahl der Öffnungen des Grundkörpers (240) und der Anzahl der Öffnungen des zylindrischen Abschnitts (140) aufweist:
2 und 2, oder 3 und 2, oder 3 und 3, oder 4 und 2, oder 4 und 3, oder 4 und 4.

11. Verbindungssystem (10) nach einem der vorangehenden Ansprüche, **dadurch gekennzeichnet, dass**
die torförmigen Durchlassöffnungen (110, 120, 130) des Stellglieds (100) einen halbrunden, insbesondere U-förmigen, rechteckigen oder dreieckigen Abschnitt aufweisen.

12. Verbindungssystem (10) nach einem der vorangehenden Ansprüche, **dadurch gekennzeichnet, dass**
die mit dem Fluid in Kontakt kommenden Oberflächen des Verbindungssystems (10) wenigstens abschnittsweise aus einem amorphen Copolyester hergestellt werden.

13. Verbindungssystem (10) nach Anspruch 12, **dadurch gekennzeichnet, dass** neben dem Copolyester weitere Materialien verwendet werden, welche aus einer Gruppe ausgewählt werden, welche duro- und thermoplastische Kunststoff und insbesondere Polyphenylensulfid, Polypropylen, Poly-1-buten, 10 Polyvinylchlorid, Polyvinylidenchlorid, Polymethyl-metaacrylat, Polyacrylnitril, Polystyrol, Polysulfon, Polyacetal, Polyvinylalkohol, Polyvinylacetat, Ionomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff- Harz, Melamin-Harz, Alkylharz, Allylharz, Silicon, Polyimid, Polybenzimidazol, Epoxidharz, Casein-Kunststoff, vernetztes Polyurethan, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen, und/oder Kombinationen hiervon umfasst.

## Claims

1. A connection system (10) for fluid-flowing components for medical and/or medical technology, having an actuator (100) having a cylindrical portion (140) with an outer surface (143),
- wherein a handle (150) is arranged at a first end (141) of the cylindrical portion (140),
- wherein the cylindrical portion (140) has a hollow space (145) in the region of a second end (142), which is open towards the second end (142), and
- wherein at least two gate-shaped passage openings (110, 120, 130) for a fluid connection are arranged in the region of the second end (142), and
a base housing (200), having a base body (240) and at least two fluid passages (210, 220, 230) arranged laterally on the base body (240),
- wherein the base body (240) has a cavity (245) with an open first end (241), a base (242) in the region of a second end and at least two laterally arranged openings (214, 224, 234), which each establish a fluid connection to the corresponding fluid passages (210, 220, 230),
- wherein the cavity (245) receives the cylindrical portion (140) of the actuator (100) in a rotatable manner through the open first end (241) and the inner diameter of the cavity (245) corresponds to the outer diameter of the cylindrical portion (140) of the actuator (100), and
- wherein the laterally arranged openings (214, 224, 234) correspond to the passage openings (110, 120, 130) of the actuator (100),
**characterised in that**
a substantially cylindrical pin (260) for supporting the second end (142) of the actuator (100) is arranged on the base (242) of the base body (240), and
a circumferential groove (165) is formed in the region of the second end (142) of the actuator (100), which groove at least partially embraces the pin (260) and thus forms a counter-bearing for the second end (142) of the actuator (100), wherein the groove (165) contacts the pin (260), and
the upper edge of the pin (260) is not higher than the lower edge of one of the fluid passages (210, 220, 230).

2. The connection system (10) according to claim 1, **characterised in that**
the pin (260) has a cavity (265).

3. The connection system (10) according to any of the preceding claims, **characterised in that**
the pin (260) has apertures (262).

4. The connection system (10) according to any of the preceding claims, **characterised in that**
the pin (260) and the corresponding circumferential fillet (165) are at least partially bevelled or rounded.

5. The connection system (10) according to any of the preceding claims, **characterised in that**
the fluid passage (210, 220, 230) of the base housing (200) has an internal thread, an external thread or another suitable connection structure in the region of its external opening (212, 222, 232) in order to act as a connection point for a component through which fluid flows, in particular for medicine and/or medical technology.

6. The connection system (10) according to any of the preceding claims, **characterised in that**
when the cylindrical portion (140) of the actuator (100) is arranged in the base body (240) of the base housing (200), a distance remains between the second end (142) of the actuator (100) and the bottom of the cavity (245), so that an annular free space (270) is formed.

7. The connection system (10) according to any of the preceding claims, **characterised in that**
the cylindrical portion (140) of the actuator (100) has an annular formation (190) and the base body (240) of the base housing (200) has a radially circumferential recess (290),
so that, when the cylindrical portion (140) of the actuator (100) is arranged in the base body (240) of the base housing (200), the annular moulding (190) engages in the radially circumferential recess (290).

8. The connection system (10) according to any of the preceding claims, **characterised in that**
the cylindrical portion (140) has a further cavity (147) in the region of a first end (141), which is separated from the cavity (145).

9. The connection system (10) according to any of the preceding claims, **characterised in that**
in the region of the second end (142) of the cylindrical portion (140), between the passage openings (110, 120, 130), a projection and/or a recess, which extends horizontally or vertically, is arranged, which corresponds to a recess and/or projection of the pin (260).

10. The connection system (10) according to any of the preceding claims, **characterised in that**
the connection system (10) preferably has the following combinations of the number of openings of the base body (240) and the number of openings of the cylindrical portion (140) :
2 and 2, or 3 and 2, or 3 and 3, or 4 and 2, or 4 and 3, or 4 and 4.

11. The connection system (10) according to any of the preceding claims, **characterised in that**
the gate-shaped passage openings (110, 120, 130) of the actuator (100) have a semicircular, in particular U-shaped, rectangular or triangular portion.

12. The connection system (10) according to any of the preceding claims, **characterised in that**
the surfaces of the connection system (10) which come into contact with the fluid are produced at least in portions from an amorphous copolyester.

13. The connection system (10) according to claim 12, **characterised in that,**
in addition to the copolyester, further materials are used which are selected from a group comprising thermosetting and thermoplastic plastics and in particular polyphenylene sulphide, polypropylene, poly-1-butene, polyvinyl chloride, polyvinylidene chloride, polymethyl metacrylate, polyacrylonitrile, polystyrene, polysulfone, polyacetal, polyvinyl alcohol, polyvinyl acetate, lonomers, fluoroplastics, polyethylene, polyamide, in particular a partially aromatic polyamide, polycarbonate, polyester, polyphenylene oxide, polysulfone, polyvinyl acetal, polyurethane, and chlorinated polyether, cellulose nitrate, cellulose acetate, cellulose ether, phenolic resin, urea resin, urea resin, thiourea resin, melamine resin, alkyl resin, allyl resin, silicone, polyimide, polybenzimidazole, epoxy resin, casein plastic, cross-linked polyurethane, unsaturated polyester resin, antimicrobial or antiseptic materials such as for example, highly porous silver, ion-free silver, silver compounds and in particular microsilver, metal ion-releasing compounds, and/or combinations thereof.

## Revendications

1. Système de raccordement (10) pour des composants traversés par un fluide utilisés en médecine et/ou dans une technique médicale, avec
un organe de réglage (100) présentant une section cylindrique (140) avec une surface externe (143),
- dans lequel une poignée (150) est disposée sur une première extrémité (141) de la section cylindrique (140),
- dans lequel la section cylindrique (140) présente dans la zone d'une deuxième extrémité (142) un espace creux (145), qui est ouvert en direction de la deuxième extrémité (142), et
- dans lequel au moins deux ouvertures de passage (110, 120, 130) en forme de porte pour un raccordement fluidique sont disposées dans la zone de la deuxième extrémité (142),
et
un boîtier de base (200), présentant un corps de base (240) et au moins deux passages de fluide (210, 220, 230) disposés latéralement sur le corps de base (240),
- dans lequel le corps de base (240) présente un espace creux (245) avec une première extrémité ouverte (241), un fond (242) dans la zone d'une deuxième extrémité et au moins deux ouvertures (214, 224, 234) disposées latéralement, lesquelles établissent respectivement un raccordement fluidique vers les passages de fluide (210, 220, 230) correspondants,
- dans lequel l'espace creux (245) reçoit de manière mobile par rotation la section cylindrique (140) de l'organe de réglage (100) par la première extrémité ouverte (241) et le diamètre intérieur de l'espace creux (245) correspond au diamètre extérieur de la section cylindrique (140) de l'organe de réglage (100), et
- dans lequel les ouvertures (214, 224, 234) disposées latéralement correspondent aux ouvertures de passage (110, 120, 130) de l'organe de réglage (100),
**caractérisé en ce que**
un tourillon (260) sensiblement cylindrique destiné à soutenir la deuxième extrémité (142) de l'organe de réglage (100) est disposé sur le fond (242) du corps de base (240), et
une gorge périphérique (165) est formée dans la zone de la deuxième extrémité (142) de l'organe de réglage (100), laquelle entoure au moins en partie le tourillon (260) et forme ainsi un contre-palier pour la deuxième extrémité (142) de l'organe de réglage (100), dans lequel la gorge (165) est en contact avec le tourillon (260), et
le bord supérieur du tourillon (260) n'est pas plus haut que le bord inférieur d'un des passages de fluide (210, 220, 230).

2. Système de raccordement (10) selon la revendication 1, **caractérisé en ce que** le tourillon (260) présente un espace creux (265).

3. Système de raccordement (10) selon l'une quelconque des revendications précédentes, **caractérisé en ce que** le tourillon (260) présente des ajours (262).

4. Système de raccordement (10) selon l'une quelconque des revendications précédentes, **caractérisé en ce que** le tourillon (260) et la gorge périphérique (165) correspondante sont chanfreinés ou arrondis au moins en partie.

5. Système de raccordement (10) selon l'une quelconque des revendications précédentes, **caractérisé en ce que** le passage de fluide (210, 220, 230) du boîtier de base (200) présente dans la zone de son ouverture extérieure (212, 222, 232) un filetage intérieur, un filetage extérieur ou une autre structure de raccordement adaptée pour faire office d'emplacement de connexion pour un composant traversé par un fluide, en particulier utilisé en médecine et/ou dans une technique médicale.

6. Système de raccordement (10) selon l'une quelconque des revendications précédentes, **caractérisé en ce que** lorsque la section cylindrique (140) de l'organe de réglage (100) est disposée dans le corps de base (240) du boîtier de base (200), un espacement entre la deuxième extrémité (142) de l'organe de réglage (100) et le fond de l'espace creux (245) demeure de telle sorte qu'un espace libre annulaire (270) est formé.

7. Système de raccordement (10) selon l'une quelconque des revendications précédentes, **caractérisé en ce que** la section cylindrique (140) de l'organe de réglage (100) présente une déformation annulaire (190) et le corps de base (240) du boîtier de base (200) présente un évidement radialement périphérique (290),
de telle sorte que, lorsque la section cylindrique (140) de l'organe de réglage (100) est disposée dans le corps de base (240) du boîtier de base (200), la déformation annulaire (190) s'enclenche dans l'évidement radialement périphérique (290).

8. Système de raccordement (10) selon l'une quelconque des revendications précédentes, **caractérisé en ce que** la section cylindrique (140) présente dans la zone d'une première extrémité (141) un autre espace creux (147), qui est séparé de l'espace creux (145).

9. Système de raccordement (10) selon l'une quelconque des revendications précédentes, **caractérisé en ce que** est disposé, dans la zone de la deuxième extrémité (142) de la section cylindrique (140), entre les ouvertures de passage (110, 120, 130), une partie faisant saillie et/ou une partie en retrait, lesquelles s'étendent horizontalement ou verticalement, lesquelles correspondent à une partie en retrait et/ou une partie faisant saillie du tourillon (260).

10. Système de raccordement (10) selon l'une quelconque des revendications précédentes, **caractérisé en ce que**
le système de raccordement (10) présente de préférence des combinaisons suivantes du nombre des ouvertures du corps de base (240) et du nombre des ouvertures de la section cylindrique (140) :
2 et 2, ou 3 et 2, ou 3 et 3, ou 4 et 2, ou 4 et 3, ou 4 et 4.

11. Système de raccordement (10) selon l'une quelconque des revendications précédentes, **caractérisé en ce que** les ouvertures de passage (110, 120, 130) en forme de porte de l'organe de réglage (100) présentent une section semi-ronde, en particulier en forme de U, rectangulaire ou triangulaire.

12. Système de raccordement (10) selon l'une quelconque des revendications précédentes, **caractérisé en ce que** les surfaces parvenant en contact avec le fluide du système de raccordement (10) sont fabriquées au moins par endroits à partir d'un copolyester amorphe.

13. Système de raccordement (10) selon la revendication 12, **caractérisé en ce que** sont utilisés outre le copolyester d'autres matériaux, lesquels sont choisis parmi un groupe, qui comprend une matière synthétique thermodurcissable ou thermoplastique et en particulier du polysulfure de phénylène, du polypropylène, du poly-1-butène, du chlorure de polyvinyle, du chlorure de polyvinylidène, du métaacrylate de polyméthyle, du polyacrylonitrile, du polystyrène, de la polysulfone, du polyacétal, de l'alcool polyvinylique, du polyacétate de vinyle, des ionomères, du plastique fluoré, du polyéthylène, du polyamide, en particulier un polyamide partiellement aromatique, du polycarbonate, du polyester, du polyoxyde de phénylène, de la polysulfone, de l'acétal polyvinylique, du polyuréthane et du polyéther chloré, du nitrate de cellulose, de l'acétate de cellulose, de l'éther de cellulose, de la résine phénolique, de la résine d'urée, de la résine de thiourée, de la résine de mélamine, de la résine alkyle, de la résine allyle, du silicone, du polyimide, du polybenzimidazole, de la résine époxy, du plastique à base de caséine, du polyuréthane réticulé, de la résine de polyester non saturée, des matériaux antimicrobiens ou antiseptiques, comme de l'argent hautement poreux, de l'argent fabriqué sans ions, des composés d'argent et en particulier du nano-argent, des composés dégageant des ions métalliques, et/ou des combinaisons de ceux-ci.
